# EUROPEAN PATENT SPECIFICATION

(11) **EP 1 902 706 B1**
(45) Date of publication and mention of the grant of the patent: **02.11.2016**
(21) Application number: 07116945.2
(22) Date of filing: 21.09.2007
(51) Int. Cl.: A61K 9/00, A61K 31/65, A61K 47/00

(54) **Stable pharmaceutical compositions of tetracyclines in solution, method for obtaining them and their uses**
Stabile pharmazeutische Tetracyclin-Zusammensetzungen in Lösungen, Verfahren zu deren Herstellung und Verwendung
Compositions pharmaceutiques stables de tétracyclines en solution, leur procédé d'obtention et leurs utilisations

(30) Priority: 25.09.2006 ES 200602416
(43) Date of publication of application: 26.03.2008
(73) Proprietor: Divasa-Farmavic, S.A., 08503 Gurb (ES)
(72) Inventor: Jutglar Sellarés, Montserrat, 08503 Gurb (ES); Ramon Pou, Gemma, 08503 Gurb (ES); Foradada Sellabona, Mercè, 08503 Gurb (ES); Caballero Gobern, Francesc, 08503 Gurb (ES)
(74) Representative: Oficina Ponti, SLP

(56) References cited:
- WO-A-94/27611
- WO-A-96/01634
- WO-A-2005/046651
- US-A- 4 081 527
- US-A- 4 126 680
- US-A- 4 376 118

## Description

### Field of art

The present invention relates to the pharmaceutical field of tetracyclines and, in particular, to stable pharmaceutical compositions of tetracyclines in solution, to the method for obtaining them and to their use individually or in combination for manufacturing a drug for humans or animals for the prevention and/or treatment of conditions against which the tetracyclines show themselves to be effective, by means of any of the acceptable administration routes for drugs.

### Background of the invention

The tetracyclines form part of a family of antibiotics used for some time now, mostly in solid forms of administration and in prepared solutions. The use of this type of antibiotics in solution form has been studied due to the advantages and fields of application it permits, such as administration by parenteral route or mass treatments for administering via drinking water. This application is of particular importance in the veterinary field, where treatments of these characteristics are usual due to the advantages involved from the productive viewpoint, due to being able to administer the antibiotic directly in the tanks or by on-line dispensing, this being a system that calls for minimum labour intervention and, therefore, a reduction of production costs.

There currently exist in the market various commercial products based on tetracyclines, especially oxytetracycline and doxycycline, formulated in solution, for both human and veterinary use. However, current studies based on improved analytical techniques have permitted the emergence of products that break down consubstantially with the formulation of tetracyclines in solution. Thus, when the doxycycline is in solution, a phenomenon of epimerisation on the carbon 4 leads to formation of the 4-epidoxycycline, an epimer with scant or nil antimicrobial activity. This phenomenon does not occur when the formulation is in solid form. The occurrence of this type of impurities means that the product in solution would be rejected by some demanding markets.

Tetracyclines, preferably doxycycline and oxytetracycline, present various mechanisms of degradation, among which are the photodegradation due to the incidence of luminous radiation, oxidation phenomena, hydrolysis and epimerisation. Although the first three are relatively easy to prevent by the use of physical and/or chemical means, epimerisation poses greater difficulties, since when the tetracycline is formulated in solution, the phenomenon described above is shown.

The 4-epimer emerges progressively from the preparation of the formulation at a higher or lower speed depending on the ingredients present therein, involving a loss of antimicrobial activity of the preparation and setting bounds on the life of the product. In antimicrobial products a reduction of activity not only results in lower therapeutic effect, but can also lead to the occurrence of resistance when therapeutic levels of drug are not attained in the target organism.

The formation of epimers involves a reduction of the life of the product, in that it degrades more rapidly, which hinders achieving a product with an acceptable life in the market, especially when it is a case of exporting the product, since the time that elapses between when it is manufactured and when it reaches its final destination can be lengthy.

It has been observed that reducing the water content to the utmost and the use of anhydrous excipients, such as glycerol, propylene glycol, 2-pyrrolidone, n-methyl-2-pyrrolidone, dimethylacetamide (DMA) and dimethylformamide (DMF), alone or in combination, while it manages to reduce this phenomenon does not prevent it, simply reduce its velocity. No clear scientific explanation is yet available of the precise reasons, since total reduction of the water in the formulation should reduce this phenomenon more significantly or remove it, and, therefore, also fails to provide a solution to the problem raised.

Patents WO2005046651, US4126680, WO9427611, US3846548, US3957980, US3674859 describe the main galenical strategies for the formulation of tetracycline, mostly directed at solubilisation of the active ingredient and physical-chemical stability of the solution, but having little or no effect on the aspect of the chemical stability of the active ingredient and none of them explicitly mention the formation of the 4-epimer or 4-6-epimer or the components that might solve this problem. Some of these patents claim methods for the preparation of stable formulations, but no reference is made to the levels of impurities, nor the characterisation thereof to be prevented in the formulation.

Various strategies have been tried in order to solve the problem of epimerisation, essentially based on reducing the speed of formation of the epimer. The most frequently used strategies includes refrigeration of the solution, which involves maintaining the product at temperatures lower than 8°C; this is a disadvantage in the case of high volumes of product, since the cold chain cannot be broken during the life of the product, in addition to the extra cost and disadvantages involved in the conservation system itself.

Another of the strategies consists of the use of certain solvents that appear to improve the stability and delay the occurrence of the products of breakdown, such as 2-pyrrolidone, n-methyl-2-pyrrolidone, polypropylene glycol, propylene glycol and dimethyl acetamide, to which salts with divalent cations can be incorporated, especially calcium and magnesium, in order to form allegedly more stable complexes with the active ingredient.

Patent WO2005046651 discloses a method for obtaining stable liquid capsules of doxycycline, although the chosen galenical strategy consists of the use of solvents of a lipidic nature, which by their nature cannot be soluble in water and, therefore, could not be used in compositions to be administered by this route. Likewise, there is no mention about the phenomena of epimerisation as the main channels of breakdown of doxycycline in solution.

It would be useful in the pharmaceutical industry to have strategies for the stabilisation of tetracycline solutions, and preferably of doxycycline, as the latter is an antibiotic that due to its nature and characteristics is used in both human and veterinary medicine, being of particular interest in the latter due to the ease involved in the application of stable solutions in mass treatments for livestock or fish-farm production and by being antibiotics very widely used in this sector.

The object of the present invention is to solve the problem of the chemical stability of tetracycline solutions, preferably solutions of doxycycline, by permitting solutions stable from a chemical point of view to be obtained, in which the emergence of phenomena of epimerisation, and particularly the formation of 4-epidoxycycline and other impurities, is enormously reduced or practically cancelled over long periods of storage of the product, without the need to keep the product under conditions of refrigeration, thus allowing the product to be stored at room temperature for periods longer than three years without the occurrence of impurities or with minimum presence thereof.

### Summarised description of the invention

The object of the present invention is to provide stable pharmaceutical compositions of tetracyclines in solution.

The present invention also has the object of providing a method for preparing said stable pharmaceutical compositions of tetracyclines in solution.

Yet another object of the present invention is the individual or collective use for the manufacture of a drug for humans or animals for the prevention or treatment of conditions for which the tetracyclines show themselves to be effective by any of the acceptable routes for the administration of drugs.

### Detailed description of the invention

A first object of the present invention relates to stable pharmaceutical compositions of tetracyclines in solution.

In order to carry out the first object of the invention a solvent or mixture of solvents is used for the purpose of obtaining stable solutions of tetracyclines, preferably those in which the formation of epimers and, more preferably, the formation of 4-epidoxycycline and 4-6-epidoxycycline is reduced in such a way as to permit a product to be obtained that is stable over a long period of time.

As noted in the background, reducing the water content to the utmost and using anhydrous excipients does not prevent the phenomenon of epimerisation, but simply reduces its speed. However, when considering the presence of water molecules in the molecule of tetracycline itself, one of the active ingredient salts most widely used for formulating solutions based on tetracyclines is the hyclate form, preferably the hyclate of doxycycline. The hyclate form is a semihydrated form and, therefore, with water present in the chemical species that makes up the active ingredient, doxycycline.

Indeed, the doxycycline hyclate contains water in a proportion of 1 molecule of water for every two of doxycycline, with an amount between 1.4% and 2.8% by weight permitted according to the European Pharmacopoeia. Although it has not been possible to prove so conclusively, the authors of the present invention believe that the influence of this proportion of water contained in the active ingredient on the stability of the formulations in solution, even where the latter are formulated based on anhydrous solvents, particularly as relates to phenomena of epimerisation, could apparently make a contribution to these phenomena, in such a way that despite eliminating any presence of water in the excipient product, this is incorporated into the active ingredient itself.

In order to determine if this contributes to these phenomena, the authors of the present invention eliminated the water contained in the molecule of the hyclate form of the tetracycline to be formulated in solution, preferably of the hyclate of doxycycline, by using agents that help to remove it, either by conversion or by neutralisation of its activity. Surprisingly, these agents not only remove the water contained in the molecule of the hyclate form, but also remove water of other provenances that might be making up part of the formulation, such as the moisture absorbed by the excipients or by the active ingredient itself without being constitutive of the molecule.

Surprisingly, the strategy followed involves a change in the direction so far followed, in which all the strategies of formulation of doxycycline have been directed at limiting or preventing the use of water in the excipient substance, but not at removing it or capturing it from the active ingredient itself.

One of the advantages of the invention is that the ingredients used for the capture or removal of the water forming part of the active ingredient belong to the group of ingredients authorised for the formulation of pharmaceutical products and are among the ingredients authorised to form part of drugs for food-producing animals, due to the food-safety implications involved in this last case. Drugs that are administered to food-producing animals for human consumption are subject to specific and restrictive regulations as regards their composition. The use of dehydrating ingredients lying outside these regulations would prevent the product from being used in the livestock sphere due to the impossibility for obtaining the corresponding official authorisation for marketing it.

In addition to solving the problem of the stability of tetracycline solutions, the present invention therefore also ensures the obtaining of a product with sufficient health guarantees both in human and veterinary medicine as regards the efficacy and safety of the product.

Another important characteristic of the invention is that, in addition to guaranteeing suitable stability of the active ingredient, the composition has to allow a proper solubilisation thereof. The difficulties of solubilisation of the tetracyclines and of doxycycline in particular are well known and have been extensively described. Excipients and solvents must be used in suitable amounts and proportions to allow an appropriate solubilisation. This fact acquires particular importance in formulations for mass treatments for administration by dissolving in drinking water, as frequently occurs in the veterinary sphere. Concentrated solutions of tetracycline are used in this case, and can contain as much as 25-30% by weight of active ingredient, although between 10 and 20% by weight is more usual, thereby making solubilisation more difficult. It is therefore necessary that the excipients used for stabilisation do not interfere with the proper solubilisation of the active ingredient and indeed facilitate solubilisation.

The stable pharmaceutical compositions of tetracyclines in solution, object of this invention, consist of solutions comprising,
- at least one tetracycline selected from chlortetracycline, minocycline, oxytetracycline, doxycycline, preferably oxytetracycline and more preferably doxycycline, or mixtures thereof, in the form of a base, hyclate, methansulphate, hydrochloride, estolate, sulphate or a pharmaceutically acceptable salt, in a proportion between 2% and 30%, preferably between 8% and 22%,
- at least one main solvent selected from polyethylene glycol 200, polyethylene glycol 300, polyethylene glycol 400, polypropylene glycol, 2-pyrrolidone, propylene glycol, n-methyl-2-pyrrolidone; glycerol, dimethylacetamide, dimethylformamide, absolute ethanol, isopropyl alcohol, butyl alcohol or mixtures thereof, preferably propylene glycol, in a proportion between 20 and 95% and preferably between 40 and 90% and,
- at least one dehydrating agent selected from ethyl acetate, acetic anhydride and absolute ethanol, or mixtures thereof, preferably ethyl acetate and more preferably acetic anhydride, in a proportion of 1 to 20%.

In the present invention, dehydrating agent is taken to mean any ingredient that exercises a function of removing or reducing the water contained in the formulation, preferably that incorporated through the excipients and, more preferably, that incorporated through the active ingredients, either as humidity or extrinsic water, or as water constituting the chemical entity that makes up the active ingredient.

Ethyl acetate and acetic anhydride are compounds that react with water to form acetic acid, in such a way that they react with the water making up the active ingredient, removing it completely from the medium.

Also known is the hygroscopicity of absolute ethanol and its capacity of combining with water in a highly stable way, resulting in a mixture with an entity of its own made up of 95% ethanol and 5% water, to the degree that the physical separation of these two substances thus mixed is not easy matter by the usual methods of separation of alcohols, such as distillation, since the mixture behaves like a single substance and the application of more complex techniques is required to separate them. Such mixtures are known by the name of azeotropes. Absolute ethanol is preferred because it acts as cosolvent and dehydrating agent at the same time.

In addition to the basic components (tetracycline, solvent and dehydrating agent), the present composition can include one or more from among cosolvents, antioxidant agents, pH regulating substances, preservative agents, organoleptic characteristics modifying agents, complex-forming agents, chelating agents, technological adjuvants and other active ingredients.

In a preferred embodiment, the composition further comprises at least one cosolvent consisting of an anhydrous alcohol selected from glycerol, butyl alcohol, isopropyl alcohol, absolute ethanol, benzyl alcohol or a mixture thereof, preferably butanol and more preferably absolute ethanol because it acts as both cosolvent and dehydrating agent, or a mixture thereof in a proportion between 2 and 40% by weight, preferably between 5 and 20% by weight and more preferably between 7 and 15% by weight.

In another embodiment, the composition further comprises at least one antioxidant agent selected from EDTA, sodium metabisulphite, sodium formaldehyde sulphoxylate, sodium citrate, or a mixture thereof, with some further acting as chelating agents improving the stability in the drinking water when the product is used, especially in hard waters, in which the stability of the active ingredient can be compromised.

In yet another embodiment, the composition further comprises at least one pH regulating substance selected from the group consisting of acetic acid, tromethamol, citric acid, sodium citrate, L-arginine, L-lysine, monoethanolamine, diethanolamine, triethanolamine, hydrochloric acid, tartaric acid, ascorbic acid and phosphoric acid or a mixture thereof, due to the improvements brought in the use of the product. Preferably, the pH regulating substance is citric acid in an amount between 2 and 25% by weight.

In another additional embodiment, the composition further comprises at least one preservative agent selected from the group consisting of benzyl alcohol, absolute ethanol, propylparaben, phenol, chlorocresol, benzalconium chloride, butylparaben, methylparaben, benzoic acid, phenylethylalcohol, sodium benzoate or mixtures thereof.

In yet another preferred embodiment, the composition further comprises at least one organoleptic characteristics modifying agent selected from the group consisting of flavour enhancers, colorants, aromatising agents or mixtures thereof.

In yet another preferred embodiment, the composition further comprises at least one complex-forming agent selected from the group consisting of magnesium oxide, magnesium chloride, calcium chloride, a pharmaceutically acceptable salt of a divalent salt or mixtures thereof.

In yet another preferred embodiment, the composition further comprises at least one chelating agent selected from the group consisting of EDTA, citric acid, sodium citrate or mixtures thereof.

In yet another preferred embodiment, the composition further comprises at least one technological adjuvant selected from the group consisting of polyvinylpyrrolidone, carboxymethylcellulose, hydroxypropylcellulose or mixtures thereof, for the purpose of modifying the viscosity, providing a delayed-release effect, improving stability in water, etc.

The present invention also finds application in compositions in which in addition to the tetracycline, the latter is formulated in combination with other active ingredients resulting in formulations with activities other than antimicrobial activity, or ones in which more than one antimicrobial can occur at the same time. In a preferred embodiment the composition therefore further comprises at least another active ingredient selected from the group consisting of an additional tetracycline, preferably doxycycline, a mucolytic, an expectorant, an antitussive, an anti-inflammatory, an antipyretic, a bronchodilator, an antihistamine, a beta-blocker and an antibiotic or mixtures thereof. In particular, the preferred active ingredients are bromhexine, acetylcysteine, bronvanexine, ambroxol, carbocysteine, guanifensine, plant extracts or derivatives, such as pinol hydrate, *hedera helix, eucaliptus globulus,* Tolú balsam, aspirin, ibuprofen, paracetamol, metamizol, flunixin meglumine, ketoprofen, naproxene, dichlofenac, codein, dextromethorphane, noscapine, dihydrocodein, dimemorphane, cloperastine, levodropizine, fominoben, Drosera Sp extract, Drosera rotundifolia tincture, ephedrine, chlorfenamine, ebastine, loratadine, cetirizine, teofiline, salbutamol, salmeterol, terbutaline, clenbuterol, bambuterol, etamifillin, carazolol, propranolol, atenolol, gentamicine, amoxicillin, tiamulin, colistine, neomicine, lincomicine, enrofloxacin, ciprofloxacin, norfloxacin, eritromicine, streptomicine, florfenicol, ceftiofur, ivermectine, closantel, levamisol and chlorsulon. More preferably, the other active ingredient is bromhexine or paracetamol.

### Method of preparation and use

By way of example and without intending to limit the invention, there follows a description of one of the possible methods of preparing the stable pharmaceutical composition, though any method that involves sequential incorporation of ingredients and dissolution thereof by means of energy provision, preferably mechanical, is valid.

The method of preparation can comprise the following steps:
a) Addition of at least one solvent in a reactor provided with stirring, and subsequent stirring for 5 minutes.
b) Addition of the active ingredient and, optionally, at least one cosolvent maintaining stirring and setting the necessary speed until total dissolution.
c) Addition of the dehydrating agent and, optionally, at least one cosolvent, maintaining the stirring mechanism.
d) Addition of the rest of the ingredients until total dissolution and homogenisation.

The solution thus prepared is packaged, preferably in hermetically sealed opaque, high-density polythene containers.

Any container mentioned in this description is mentioned only by way of example, being useful for this invention any kind of packaging that can contain it.

Normally in the preparation 30°C is not exceeded, especially after adding the tetracycline; however, and especially when the formulation is a combination with other or others active ingredient(s), methods of solubilisation thereof can be used, by means of thermal or mechanical energy.

Preferably, for the purpose of improving the incorporation of ingredients, prior mixtures thereof can be prepared in the cosolvents or in the solvent in which they are more soluble, and then once premixed they can be added to the rest of the formulation.

In a particular embodiment, the other active ingredient to be added is selected from paracetamol and bromhexine and is added following dissolution in the cosolvent or in a sufficient amount of the main solvent.

Where the preparation is for a formulation to be administered parenterally, the necessary operations are carried out in order to maintain an absence of microorganisms in the end product, whether by aseptic preparation with the ingredients sterilised beforehand, by filtering the primary solution, or by final sterilisation or any of the methods pharmaceutically acceptable for the preparation of parenteral solutions.

As regards the routes of administration, the stable pharmaceutical composition of tetracyclines in solution can be administered by oral, parenteral, intranasal, rectal, inhalatory, transcutaneous, topical, ocular, intramuscular, subcutaneous, intravenous, intradermal, intratecal and intramammary routes, or any other route acceptable for the administration of drugs in human or veterinary medicine. Said administration can be carried out through water, by direct dissolution, with the help of automatic dispensing systems, on-line dispensing systems, by means of mechanical stirring agents or with any acceptable method or system of dispensing.

The pharmaceutical composition of the present invention can be administered to individuals of the human, porcine, bovine, equine, canine, feline species and to birds, rodents, reptiles, poultry, cage birds, marine animals, freshwater animals and ornamental fish. It can additionally be administered to livestock and for the treatment of freshwater or saltwater fish farms.

Another aspect of the present invention relates to the use of said stable pharmaceutical composition of tetracyclines in solution for the manufacturing of a drug for the prevention and/or treatment of a respiratory, dermatological, digestive, circulatory, articular or tissular pathology of any ethology for which active ingredients of the formulation show themselves to be effective.

By way of non-restrictive illustration, there follow descriptions of some examples of compositions of the present invention.

### Examples of the composition:

### Example 1: Uncombined formulations of tetracycline

| **Ingredients** | **A** | **B** | **C** |
|---|---|---|---|
| Doxycycline hyclate | 11.350 g | 11.350 g | 22.700g |
| N-methyl-2-pyrrolidone | 5.000 g | 0.005 g | 8.000 g |
| Polyethylene glycol 300 | 5.000 g | 0.001 g | 4.0.00 g |
| Absolute ethanol | 7.000 g | 10.000 g | 6.000 g |
| Acetic anhydride | 4.000 g | 0.001 g | 1.000 g |
| Ethyl acetate | 1.000 g | 0.001 g | 0.001 g |
| Sodium formaldehyde sulphoxylate | 0.400 g | 0.001 g | 0.001 g |
| MnO | 0.800 g | 0.001 g | 0.001 g |
| Propylene glycol | q.s. 100 ml | q.s. 100 ml | q.s. 100 ml |

### Example 2: Tetracycline solutions combined with mucolytics

| **Ingredients** | **A** | **B** | **C** |
|---|---|---|---|
| Doxycycline hyclate | 22.600 | 11.600 g | 16.400 g |
| Bromhexine.hydrochloride | 1.000 g | 0.000 g | 1.000 g |
| n-acetylcysteine | 0.000 g | 2.000 g | 0.000 g |
| Carbocysteine | 0.000 g | 1.000 g | 0.000 g |
| N-methyl-2-pyrrolidone | 3.000 g | 0.005 g | 0.001 g |
| N-N-dimethylacetamide | 1.000 g | 0.000 g | 0.005 g |
| Poliethylene glycol 300 | 2.000 g | 0.001 g | 0.001 g |
| Absolute ethanol | 7.000 g | 7.000 g | 10.000 g |
| Acetic anhydride | 0.500 g | 3.000 g | 0.001 g |
| Ethyl acetate | 0.001 g | 0.001 g | 0.001 g |
| Sodium formaldehyde sulphoxylate | 0.400 g | 0.001 g | 0.001 g |
| Acetic acid | 0.001 g | 4.000 g | 0.005 g |
| Citric acid | 8.000 g | 6.000 g | 10.000 g |
| Propylene glycol | q.s. 100 ml | q.s. 100 ml | q.s. 100 ml |

### Example 3: Tetracycline solutions combined with antipyretics

| **Ingredients** | **A** | **B** | **C** |
|---|---|---|---|
| Doxycycline hyclate | 11.600 g | 11.600 g | 11.600 g |
| Flunixin meglumin | 5.000 g | 0.000 g | 0.000 g |
| Aspirin | 0.000 g | 20.000 g | 0.000 g |
| Paracetamol | 0.000 g | 0.000 g | 20.000 g |
| N-methyl-2-pyrrolidone | 5.000 g | 0.005 g | 2.000 g |
| N-N-dimethylacetamide | 3.000 g | 20.000 g | 0.001 g |
| Polyethylene glycol 300 | 25.000 g | 0.001 g | 5.000 g |
| Absolute ethanol | 9.000 g | 10.000 g | 15.000 g |
| Ethyl acetate | 0.000 g | 0.001 g | 0.001 g |
| Sodium formaldehyde sulphoxylate | 0.001 g | 0.001 g | 0.010 g |
| Propylene glycol | q.s. 100 ml | q.s. 100 ml | q.s. 100 ml |

### Evaluation of stability

The chemical stability of the formulations of the examples was studied. The stability study was carried out at the analysis facilities of the R&D Department of the Divasa-Farmavic, S.A. laboratories.

A laboratory batch was prepared for the study following the formulation pattern of the compositions of the examples, and its content in doxycycline and impurities was analysed by means of high-resolution liquid chromatography with UV-VIS detector, using a method previously validated for the exact and precise determination of the active ingredient doxycycline and of all the impurities, based on the method described by Yekkala and col., Chromatography, 2003. The identification of the chromatographic peaks was carried out by checking the retention times and by comparing their UV-VIS spectra by means of a sweeping at different wavelengths using a diode detector. The following reference solutions were used, prepared in 0.01 M HCl: 5% 4-epidoxycycline, 35% metacycline and 20% 6-epidoxycycline. The last two solutions were subjected to conditions of stress for obtaining epimetacycline and 4,6 epidoxycycline.

The chromatographic system used was based on an adaptation for the finished product of the technique described in the European Pharmacopoeia, in order to separate properly the various impurities.

In the technique used, the typical chromatogram showed the following retention times. Doxycycline (21.50 min), metacycline (18.19 min), 4-epidoxycycline (6.99 min), 6-epidoxiciclione (15.39 min), 4,6 epidoxycycline + epimetacycline (10.86 min), while the rest of the peaks were considered to be unknown impurities.

The preparations were packaged in hermetically sealed high-density polyethylene containers inside regulated-temperature and -humidity climatic chambers, under the following conditions:
Condition a) at 25°C 60% relative humidity
Condition b) at 40 °C 75% relative humidity
Condition c) refrigerator T< 8°C

The stored preparations were analysed at intervals, the last determination being at 3 years at 25°C and 8°C and 12 months at 40°C.

A reference sample, without any dehydrating agents, other commercial formulations and a placebo solution without doxycycline were also prepared for the study in order to have positive and negative controls for the comparison of results.

The European Pharmacopoeia sets the limits for 6-epidoxycycline and metacycline at a maximum of 2% by weight, while any other impurity must not exceed 0.5% by weight. The United States Pharmacopoeia (USP), sets the same limits for all impurities as the European Pharmacopoeia, except for metacycline, which has no specific limit set.

The results obtained show a minimal disappearance of the doxycycline peak, indicating that there had been no degradation or transformation of the molecule over the period of study under the storage conditions. A scant presence of 4-epidoxycycline was also revealed following 3 years' storage, in relation to the formulations used as reference, with a clear peak corresponding to the epimer, as well as a significant reduction of the doxycycline content in the samples used as positive references.

The samples stored at higher temperature did not show significance occurrence of the epimer either, thus reinforcing the results obtained in condition a), since temperature acts as an accelerator of the degradation reactions by acting to simulate the passage of time.

The samples of formulations prepared without the desiccant and/or the alcohol, especially absolute ethyl alcohol, showed themselves to be unstable within only a few weeks of preparation thereof, with the 4-epimer starting to be increasingly and significantly detectable over the course of the study.

The chromatograms corresponding to the reference formulations revealed an apparent peak corresponding to 4-epidoxycycline over the course of the storage, resulting in a compensation of the 4-epidoxycycline peak due to a loss of the active form. The mass balance, taking into account all the components except for the unknown impurities, resulted in 102.3%, involving that the unknown impurities are probably not related with the doxycycline.

There follow below the results corresponding to preparations that followed the model of formulation 2A and 1B (6-EDOX = 6-epidoxycycline; 4-EDOX = 4-epidoxycycline; OTHER IMP = metacycline + 4,6-epidoxycycline + epimetacycline + unknown impurities).

**Table 1: Results of the formulation of example 2-A Conditions: refrigerator**

| **Time (month)** | **Doxycycline content (mg/ml)** | **% compared with initial** | **4-EDOX mg/ml** |
|---|---|---|---|
| **0** | 201.89 | 100.00 | 0.000 |
| **1** | 200.79 | 99.45 | 0.000 |
| **2** | 204.63 | 101.36 | 0.000 |
| **3** | 206.77 | 102.42 | 0.000 |
| **6** | 204.70 | 101.39 | 0.000 |
| **12** | 205.67 | 101.87 | 0.000 |
| **24** | 201.96 | 100.04 | 0.005 |
| **36** | 200.507 | 99.32 | 0.041 |

**Table 2: Results of the formulation of example 2-A Conditions: 25°C - 60% RH**

| **Time (month)** | **Doxycycline content (mg/ml)** | **% compared with initial** | **4-EDOX mg/ml** |
|---|---|---|---|
| **0** | 201,89 | 100,00 | 0,000 |
| **1** | 203.02 | 100.56 | 0.000 |
| **2** | 206.42 | 102.25 | 0.027 |
| **3** | 209.04 | 103.54 | 0.259 |
| **6** | 205.32 | 101.70 | 0.516 |
| **12** | 203.41 | 100.75 | 0.823 |
| **24** | 202.46 | 99.74 | 1.115 |
| **36** | 194.72 | 96.45 | 3.433 |

**Table 3: Results of the formulation of example 2-A Conditions: 40°C - 75% RH**

| **Time (month)** | **Doxycycline content (mg/ml)** | **% compared with initial** | **4-EDOX mg/ml** |
|---|---|---|---|
| **0** | 201.89 | 100.00 | 0.000 |
| **1** | 204.56 | 101.32 | 0.633 |
| **2** | 202.98 | 100.54 | 1.354 |
| **3** | 203.62 | 100.86 | 2.749 |
| **6** | 196.31 | 97.24 | 3.932 |
| **12** | 195.93 | 97.05 | 4.874 |

**Table 4: Results of the formulation of example 1-B Conditions: refrigerator <8°C**

| **Time (month)** | **Doxycycline content (mg/ml)** | **% compared with initial** | **4-EDOX mg/ml** |
|---|---|---|---|
| **0** | 101.42 | 100.00 | 0.000 |
| **8** | 101.86 | 100.43 | 0.032 |
| **15** | 100.04 | 99.63 | 0.118 |
| **24** | 101.14 | 99.72 | 0.322 |
| **36** | 100.61 | 99.20 | 0.524 |

**Table 5: Results of the formulation of example 1-B Conditions: 25°C - 65% RH**

| **Time (month)** | **Doxycycline content (mg/ml)** | **% compared with initial** | **4-EDOX mg/ml** |
|---|---|---|---|
| **0** | 101.42 | 100.00 | 0.000 |
| **8** | 101.12 | 99.70 | 0.319 |
| **15** | 100.19 | 98.79 | 0.755 |
| **24** | 99.02 | 97.63 | 0.902 |
| **36** | 96.79 | 95.44 | 2.175 |

**Table 6: Results of the formulation of example 1-B Conditions: 45°C = 75% RH**

| **Time (month)** | **Doxycycline content (mg/ml)** | **% compared with initial** | **4-EDOX mg/ml** |
|---|---|---|---|
| **0** | 101.42 | 100.00 | 0.000 |
| **6** | 98.28 | 96.90 | 2.633 |

**Table 7: Results of the reference formulation under Conditions: 25°C - 65% RH**

| **Time (month)** | **Doxycycline content (mg/ml)** | **% compared with initial** | **4-EDOX mg/ml** |
|---|---|---|---|
| **0** | 100.12 | 100 | 0.000 |
| **15** | 88.83 | 88.72 | 10.941 |

The results of the study carried out show that:
- the incorporation of dehydrating agents into the formulations that contain active ingredients in the form of hyclate and are liable to degrade in the presence of water improves the stability of said active ingredients in the final formulation.
- the incorporation of dehydrating agents into the formulations of doxycycline in solution improves their stability by preventing the ocurrence of impurities and related substances.
- the incorporation of dehydrating agents into the formulations of doxycycline in solution prevents epimerisation reactions, and specifically the formation of 4-epidoxycycline.

## Claims

1. Pharmaceutical composition in solution form comprising:
- at least one tetracycline selected from the group consisting of chlortetracycline, minocycline, oxytetracycline, doxycycline in a proportion between 2 and 30% by weight,
- at least one solvent selected from the group consisting of polyethylene glycol 200, polyethylene glycol 300, polyethylene glycol 400, polypropylene glycol, 2-pyrrolidone, propylene glycol, n-methyl-2-pyrrolidone, glycerol, dimethylacetamide, dimethylformamide, absolute ethanol, isopropyl alcohol, butyl alcohol, in a proportion between 20 and 95% by weight, except for water, and
- at least one dehydrating agent in a proportion between 1 and 20% by weight.

2. Pharmaceutical composition according to claim 1, **characterised in that** said tetracycline is in a proportion between 8 and 22% by weight.

3. Pharmaceutical composition according to claim 1, **characterised in that** said solvent is in a proportion between 40 and 90% by weight.

4. Pharmaceutical composition according to claims 1 or 2, **characterised in that** said tetracycline is in the form of a base, hyclate, methansulphate, hydrochloride, estolate, sulphate or a pharmaceutically acceptable salt.

5. Pharmaceutical composition according to any of the preceding claims, **characterised in that** said tetracycline is doxycycline.

6. Pharmaceutical composition according to claim 1 or 3, **characterised in that** said solvent is propylene glycol.

7. Pharmaceutical composition according to claim 1, **characterised in that** said dehydrating agent is selected from the group consisting of absolute ethanol, ethyl acetate and acetic anhydride.

8. Pharmaceutical composition according to claim 7, **characterised in that** said dehydrating agent is absolute ethanol.

9. Pharmaceutical composition according to any of the preceding claims, **characterised in that** it further comprises at least one cosolvent selected from the group consisting of glycerol, butyl alcohol, isopropyl alcohol, absolute ethanol and benzyl alcohol.

10. Pharmaceutical composition according to claim 9, **characterised in that** said cosolvent is in a proportion between 2 and 40% by weight.

11. Pharmaceutical composition according to claim 10, **characterised in that** said cosolvent is in a proportion between 5 and 20% by weight.

12. Pharmaceutical composition according to claim 10, **characterised in that** said cosolvent is in a proportion between 7 and 15% by weight.

13. Pharmaceutical composition according to any of the preceding claims, **characterised in that** it further comprises at least one pH regulating substance selected from the group consisting of acetic acid, trometamol, citric acid, sodium citrate, L-arginine, L-lysine, monoethanolamine, diethanolamine, triethanolamine, hydrochloric acid, tartaric acid, ascorbic acid and phosphoric acid.

14. Pharmaceutical composition according to claim 13, **characterised in that** said pH regulating substance is citric acid.

15. Pharmaceutical composition according to any of the preceding claims, **characterised in that** it further comprises at least one antioxidant agent selected from the group consisting of EDTA, sodium metabisulphite and sodium formaldehyde sulphoxylate.

16. Pharmaceutical composition according to any of the preceding claims, **characterised in that** it further comprises at least one preservative agent selected from the group consisting of benzyl alcohol, absolute ethanol, propylparaben, phenol, chlorocresol, benzalconium chloride, butyliparaben, methylparaben, benzoic acid, phenylethylalcohol and sodium benzoate.

17. Pharmaceutical composition according to any of the preceding claims, **characterised in that** it further comprises at least one organoleptic characteristics modifying agent, selected from the group consisting of flavour enhancers, colorants and aromatising agents.

18. Pharmaceutical composition according to any of the preceding claims, **characterised in that** it further comprises at least one complex-forming agent selected from the group consisting of magnesium oxide, magnesium chloride, calcium chloride and a pharmaceutically acceptable salt of a divalent cation.

19. Pharmaceutical composition according to any of the preceding claims, **characterised in that** it further comprises at least one chelating agent selected from the group consisting of EDTA, citric acid and sodium citrate.

20. Pharmaceutical composition according to any of the preceding claims, **characterised in that** it further comprises at least one technological adjuvant selected from the group consisting of polyvinylpyrrolidone, carboxymethylcellulose and hydroxypropylcellulose.

21. Pharmaceutical composition according to any of the preceding claims, **characterised in that** it further comprises at least another active ingredient selected from the group consisting of a mucolytic, an expectorant, an antitussive, an anti-inflammatory, an antipyretic, a bronchodilator, an antihistamine, a beta-blocker and an antibiotic.

22. Pharmaceutical composition according to claim 21, **characterised in that** said another active ingredient is selected from the group consisting of bromhexine, acetylcysteine, bronvanexine, ambroxol, carbocysteine, guanifensine, plant extracts or derivatives, such as pinol hydrate, *hedera helix, eucaliptus globulus,* Tolú balsam, aspirin, ibuprofen, paracetamol, metamizol, flunixin meglumine, ketoprofen, naproxene, dichlofenac, codein, dextrometorphane, noscapine, dihydrocodein, dimemorphane, cloperastine, levodropizine, fominoben, Drosera Sp extract, Drosera rotundifolia tincture, ephedrine, chlorfenamine, ebastine, loratadine, cetirizine, teofiline, salbutamol, salmeterol, terbutaline, clenbuterol, bambuterol, etamifillin, carazolol, propranolol, atenolol, gentamicine, amoxicillin, tiamulin, colistine, neomicine, lincomicine, enrofloxacin, ciprofloxacin, norfloxacin, eritromicine, streptomicine, florfenicol, ceftiofur, ivermectine, closantel, levamisol and chlorsulon.

23. Pharmaceutical composition according to claim 22, **characterised in that** said other active ingredient is bromhexine.

24. Pharmaceutical composition according to claim 22, **characterised in that** said other active ingredient is paracetamol.

25. Pharmaceutical composition according to any of the preceding claims, **characterised in that** it is administered by oral, parenteral, intranasal, rectal, inhalatory, transcutaneous, topical, ocular, intramuscular, subcutaneous, intravenous, intradermal, intratecal and intramammary routes.

26. Method for obtaining a pharmaceutical composition, according to any of the preceding claims, **characterised in that** it comprises the steps of:
a) Addition of at least one solvent in a reactor provided with stirring, and subsequent stirring.
b) Addition of an active ingredient and, optionally, at least one cosolvent, maintaining stirring and setting the necessary speed until total dissolution.
c) Addition of a dehydrating agent and, optionally, at least one cosolvent, maintaining the stirring mechanism.
d) Addition of the rest of the ingredients until total dissolution and homogenisation
in which said solvent, said active ingredient, said dehydrating agent and said cosolvent are as defined in the preceding claims.

27. Method according to claim 26, **characterised in that** said rest of the ingredients are selected from the group consisting of a pH regulating substance, antioxidant agent, preservative agent, organoleptic characteristics modifying agent, complex-forming agent, chelating agent, technological adjuvant and another active ingredient.

28. Method according to claim 27, **characterised in that** said other active ingredient is selected from paracetamol and bromhexine and are added following dissolution in the cosolvent or in a sufficient amount of the main solvent.

29. Use of a pharmaceutical composition, according to any of claims 1-25, for the manufacture of a drug for the prevention and/or treatment of a respiratory, dermatological, digestive, circulatory, articular or tissular pathology of any ethology, for which the active ingredients of the formulation show themselves to be effective.

30. Use of a pharmaceutical composition according to claim 29, **characterised in that** said administration is for individuals of the human, porcine, bovine, equine, canine, feline species and birds, rodents, reptiles, poultry, cage birds, marine animals, freshwater animals and ornamental fish.

31. Use of a pharmaceutical composition according to claim 29, **characterised in that** said administration is for livestock, treatment of freshwater or saltwater fish farms.

32. Use of a pharmaceutical composition according to any of claims 29-31, **characterised in that** said administration is carried out by means of water, direct dissolution, with the aid of automatic dispensing systems, on-line dispensing systems, mechanical stirring agents or with any acceptable method or system of dispensing.

## Patentansprüche

1. Pharmazeutische Zusammensetzung in Lösungsform, die folgende Merkmale aufweist:
zumindest ein Tetracyclin, das aus der aus Chlortetracyclin, Minocyclin, Oxytetracyclin, Doxycyclin bestehenden Gruppe ausgewählt ist, in einem Anteil zwischen 2 und 30 Gew.-%,
zumindest ein Lösungsmittel, das aus der aus Polyethylenglykol 200, Polyethylenglykol 300, Polyethylenglykol 400, Polypropylenglykol, 2-Pyrrolidon, Propylenglykol, n-Methyl-2-Pyrrolidon, Glycerol, Dimethylacetamid, Dimethlyformamid, absolutem Alkohol, Isopropylalkohol, Butylalkohol bestehenden Gruppe ausgewählt ist, in einem Anteil zwischen 20 und 95 Gew.-%, mit Ausnahme von Wasser, und
zumindest ein Wasser entziehendes Mittel in einem Anteil zwischen 1 und 20 Gew.-%.

2. Pharmazeutische Zusammensetzung gemäß Anspruch 1, **dadurch gekennzeichnet, dass** das Tetracyclin in einem Anteil zwischen 8 und 22 Gew.-% vorliegt.

3. Pharmazeutische Zusammensetzung gemäß Anspruch 1, **dadurch gekennzeichnet, dass** das Lösungsmittel in einem Anteil zwischen 40 und 90 Gew.-% vorliegt.

4. Pharmazeutische Zusammensetzung gemäß Anspruch 1 oder 2, **dadurch gekennzeichnet, dass** das Tetracyclin in Form einer Base, eines Hyclats, Methansulfats, Hydrochlorids, Estolats, Sulfats oder eines pharmazeutisch akzeptablen Salzes vorliegt.

5. Pharmazeutische Zusammensetzung gemäß einem der vorhergehenden Ansprüche, **dadurch gekennzeichnet, dass** das Tetracyclin Doxycyclin ist.

6. Pharmazeutische Zusammensetzung gemäß Anspruch 1 oder 3, **dadurch gekennzeichnet, dass** das Lösungsmittel Propylenglykol ist.

7. Pharmazeutische Zusammensetzung gemäß Anspruch 1, **dadurch gekennzeichnet, dass** das Wasser entziehende Mittel aus der aus absolutem Alkohol, Ethylacetat und Essigsäureanhydrid bestehenden Gruppe ausgewählt ist.

8. Pharmazeutische Zusammensetzung gemäß Anspruch 7, **dadurch gekennzeichnet, dass** das Wasser entziehende Mittel absoluter Alkohol ist.

9. Pharmazeutische Zusammensetzung gemäß einem der vorhergehenden Ansprüche, **dadurch gekennzeichnet, dass** sie ferner zumindest ein Hilfslösungsmittel aufweist, das aus der aus Glycerol, Butylalkohol, Isopropylalkohol, absolutem Alkohol und Benzylalkohol bestehenden Gruppe ausgewählt ist.

10. Pharmazeutische Zusammensetzung gemäß Anspruch 9, **dadurch gekennzeichnet, dass** das Hilfslösungsmittel in einem Anteil zwischen 2 und 40 Gew.-% vorliegt.

11. Pharmazeutische Zusammensetzung gemäß Anspruch 10, **dadurch gekennzeichnet, dass** das Hilfslösungsmittel in einem Anteil zwischen 5 und 20 Gew.-% vorliegt.

12. Pharmazeutische Zusammensetzung gemäß Anspruch 10, **dadurch gekennzeichnet, dass** das Hilfslösungsmittel in einem Anteil zwischen 7 und 15 Gew.-% vorliegt.

13. Pharmazeutische Zusammensetzung gemäß einem der vorhergehenden Ansprüche, **dadurch gekennzeichnet, dass** sie ferner zumindest eine den pH-Wert regelnde Substanz aufweist, die aus der aus Essigsäure, Trometamol, Zitronensäure, Natriumcitrat, L-Arginin, L-Lysin, Monoethanolamin, Diethanolamin, Triethanolamin, Chlorwasserstoffsäure, Weinsäure, Ascorbinsäure und Phosphorsäure bestehenden Gruppe ausgewählt ist.

14. Pharmazeutische Zusammensetzung gemäß Anspruch 13, **dadurch gekennzeichnet, dass** die den pH-Wert regelnde Substanz Zitronensäure ist.

15. Pharmazeutische Zusammensetzung gemäß einem der vorhergehenden Ansprüche, **dadurch gekennzeichnet, dass** sie ferner zumindest ein Antioxidationsmittel aufweist, das aus der aus EDTA, Natriummetabisulfit und Natriumformaldehydsulfoxylat bestehenden Gruppe ausgewählt ist.

16. Pharmazeutische Zusammensetzung gemäß einem der vorhergehenden Ansprüche, **dadurch gekennzeichnet, dass** sie ferner zumindest ein Konservierungsmittel aufweist, das aus der aus Benzylalkohol, absolutem Alkohol, Propylparaben, Phenol, Clorkresol, Benzalkoniumchlorid, Butylparaben, Methylparaben, Benzoesäure, Phenylethylalkohol und Natriumbenzoat bestehenden Gruppe ausgewählt ist.

17. Pharmazeutische Zusammensetzung gemäß einem der vorhergehenden Ansprüche, **dadurch gekennzeichnet, dass** sie ferner zumindest ein Mittel zur Modifizierung organoleptischer Eigenschaften aufweist, das aus der aus Geschmacksverstärkern, Farbstoffen und Aromastoffen bestehenden Gruppe ausgewählt ist.

18. Pharmazeutische Zusammensetzung gemäß einem der vorhergehenden Ansprüche, **dadurch gekennzeichnet, dass** sie ferner zumindest einen komplexbildenden Stoff aufweist, der aus der aus Magnesiumoxid, Magnesiumchlorid, Calciumchlorid und einem pharmazeutisch akzeptablen Salz eines zweiwertigen Kations bestehenden Gruppe ausgewählt ist.

19. Pharmazeutische Zusammensetzung gemäß einem der vorhergehenden Ansprüche, **dadurch gekennzeichnet, dass** sie ferner zumindest einen Chelatbildner aufweist, der aus der aus EDTA, Zitronensäure und Natriumcitrat bestehenden Gruppe ausgewählt ist.

20. Pharmazeutische Zusammensetzung gemäß einem der vorhergehenden Ansprüche, **dadurch gekennzeichnet, dass** sie ferner zumindest ein technologisches Hilfsmittel aufweist, das aus der aus Polyvinylpyrrolidon, Carboxymethylcellulose und Hydroxypropylcellulose bestehenden Gruppe ausgewählt ist.

21. Pharmazeutische Zusammensetzung gemäß einem der vorhergehenden Ansprüche, **dadurch gekennzeichnet, dass** sie ferner zumindest einen weiteren Wirkstoff aufweist, der aus der aus einem Mukolytikum, einem Expektorans, einem Antitussivum, einem Entzündungshemmer, einem Antipyretikum, einem Bronchodilatator, einem Antihistamin, einem Betablocker und einem Antibiotikum bestehenden Gruppe ausgewählt ist.

22. Pharmazeutische Zusammensetzung gemäß Anspruch 21, **dadurch gekennzeichnet, dass** der weitere Wirkstoff aus der Gruppe ausgewählt ist, die aus Bromhexin, Acetylcystein, Bronvanexin, Ambroxol, Carbocystein, Guanifensin, Pflanzenextrakten oder -derivaten, beispielsweise Pinolhydrat, *hedera helix, eucaliptus globulus,* Tolü-Balsam, Aspirin, Ibuprofen, Paracetamol, Metamizol, Flunixin-Meglumin, Ketoprofen, Naproxen, Dichlofenac, Codein, Dextromethorphan, Noscapin, Dihydrocodein, Dimemorphan, Cloperastin, Levodropizin, Fominoben, Extrakt von Drosera Sp., Tinktur von Drosera rotundifolia, Ephedrin, Chlorfenamin, Ebastin, Loratadin, Cetirizin, Teofilin, Salbutamol, Salmeterol, Terbutalin, Clenbuterol, Bambuterol, Etamifillin, Carazolol, Propranolol, Atenolol, Gentamicin, Amoxicillin, Tiamulin, Colistin, Neomicin, Lincomicin, Enrofloxacin, Ciprofloxacin, Norfloxacin, Eritromicin, Streptomicin, Florfenicol, Ceftiofur, Ivermectin, Closantel, Levamisol und Chlorsulon besteht.

23. Pharmazeutische Zusammensetzung gemäß Anspruch 22, **dadurch gekennzeichnet, dass** der weitere Wirkstoff Bromhexin ist.

24. Pharmazeutische Zusammensetzung gemäß Anspruch 22, **dadurch gekennzeichnet, dass** der weitere Wirkstoff Paracetamol ist.

25. Pharmazeutische Zusammensetzung gemäß einem der vorhergehenden Ansprüche, **dadurch gekennzeichnet, dass** sie oral, parenteral, intranasal, rektal, über die Atmungsorgane, transkutan, lokal, über die Augen, intramuskulär, subkutan, intravenös, intradermal, intrathekal und intramammär verabreicht wird.

26. Verfahren zum Erhalten einer pharmazeutischen Zusammensetzung gemäß einem der vorhergehenden Ansprüche, **dadurch gekennzeichnet, dass** es folgende Schritte aufweist:
a) Hinzufügen zumindest eines Lösungsmittels in einen mit Rühren versehenen Reaktor und anschließendes Rühren.
b) Hinzufügen eines Wirkstoffs und optional zumindest eines Hilfslösungsmittels, Beibehalten des Rührens und Einstellen der nötigen Geschwindigkeit bis zur vollständigen Auflösung.
c) Hinzufügen eines Wasser entziehenden Mittels und optional zumindest eines Hilfslösungsmittels unter Beibehaltung des Rührmechanismus.
d) Hinzufügen der restlichen Inhaltsstoffe bis zur vollständigen Auflösung und Homogenisierung
wobei das Lösungsmittel, der Wirkstoff, das Wasser entziehende Mittel und das Hilfslösungsmittel der Definition in den vorhergehenden Ansprüchen entsprechen.

27. Verfahren gemäß Anspruch 26, **dadurch gekennzeichnet, dass** die restlichen Inhaltsstoffe aus der Gruppe ausgewählt sind, die aus einer den ph-Wert regelnden Substanz, einem Antioxidationsmittel, einem Konservierungsmittel, einem Mittel zur Modifizierung organoleptischer Eigenschaften, einem komplexbildenden Stoff, einem Chelatbildner, einem technologischen Hilfsmittel und einem weiteren Wirkstoff besteht.

28. Verfahren gemäß Anspruch 27, **dadurch gekennzeichnet, dass** der weitere Wirkstoff aus Paracetamol und Bromhexin ausgewählt wird und im Anschluss an eine Auflösung in dem Hilfslösungsmittel oder in einer ausreichenden Menge des Hauptlösungsmittels hinzugegeben wird.

29. Verwendung einer pharmazeutischen Zusammensetzung gemäß einem der Ansprüche 1 bis 25 zur Herstellung eines Medikaments zur Verhinderung und/oder Behandlung einer die Atmung betreffenden, dermatologischen, die Verdauung betreffenden, den Kreislauf betreffenden, ein Gelenk oder Gewebe betreffenden Pathologie jeglicher Ethologie, für die sich die Wirkstoffe der Formulierung als wirksam erweisen.

30. Verwendung einer pharmazeutischen Zusammensetzung gemäß Anspruch 29, **dadurch gekennzeichnet, dass** die Verabreichung für Individuen der Spezies Mensch, Schwein, Rind, Pferd, Hund, Katze sowie für Vögel, Nagetiere, Reptilien, Geflügel, Käfigvögel, Meerestiere, Süßwassertiere und Zierfische gedacht ist.

31. Verwendung einer pharmazeutischen Zusammensetzung gemäß Anspruch 29, **dadurch gekennzeichnet, dass** die Verabreichung für Viehbestände, zur Behandlung von Süßwasser- oder Meerwasserfischfarmen gedacht ist.

32. Verwendung einer pharmazeutischen Zusammensetzung gemäß einem der Ansprüche 29 bis 31, **dadurch gekennzeichnet, dass** die Verabreichung anhand von Wasser, direkter Auflösung, mithilfe automatischer Abgabesysteme, Online-Abgabesystemen, mechanischen Rührmitteln oder mit einem beliebigen akzeptablen Abgabeverfahren oder -system durchgeführt wird.

## Revendications

1. Composition pharmaceutique sous la forme de solution comprenant :
- au moins une tétracycline choisie dans le groupe constitué de chlortétracycline, minocycline, oxytétracycline, doxycycline dans des proportions allant de 2 à 30% en poids,
- au moins un solvant choisi dans le groupe constitué de polyéthylène glycol 200, polyéthylène glycol 300, polyéthylène glycol 400, polypropylène glycol, 2-pyrrolidone, propylène glycol, n-méthyl-2-pyrrolidone, glycérol, diméthylacétamide, diméthylformamide, éthanol absolu, alcool isopropylique, alcool butylique dans des proportions allant de 20 à 95% en poids, à l'exception de l'eau, et
- au moins un agent déshydratant dans des proportions comprises entre 1 et 20% en poids.

2. Composition pharmaceutique selon la revendication 1, **caractérisée en ce que** ladite tétracycline est présente dans des proportions allant de 8 à 22% en poids.

3. Composition pharmaceutique selon la revendication 1, **caractérisée en ce que** ledit solvant est présent dans des proportions allant de 40 à 90% en poids.

4. Composition pharmaceutique selon la revendication 1 ou 2, **caractérisée en ce que** ladite tétracycline est présente sous forme de base, hyclate, méthansulphate, chlorhydrate, estolate, sulfate ou un sel pharmaceutiquement acceptable.

5. Composition pharmaceutique selon une quelconque des revendications précédentes, **caractérisée en ce que** ladite tétracycline est de la doxycycline.

6. Composition pharmaceutique selon la revendication 1 ou 3, **caractérisée en ce que** ledit solvant est du propylène glycol.

7. Composition pharmaceutique selon la revendication 1, **caractérisée en ce que** ledit agent déshydratant est choisi dans le groupe constitué d'éthanol absolu, d'acétate d'éthyle et d'anhydride acétique.

8. Composition pharmaceutique selon la revendication 7, **caractérisée en ce que** ledit agent déshydratant est un éthanol absolu.

9. Composition pharmaceutique selon une quelconque des revendications précédentes, **caractérisée en ce qu'**elle comprend en outre au moins un cosolvant choisi dans le groupe constitué de glycérol, alcool butylique, alcool isopropylique, éthanol absolu et alcool benzylique.

10. Composition pharmaceutique selon la revendication 9, **caractérisée en ce que** ledit cosolvant est présent dans des proportions allant de 2 à 40% en poids.

11. Composition pharmaceutique selon la revendication 10, **caractérisée en ce que** ledit cosolvant est présent dans des proportions allant de 5 à 20% en poids.

12. Composition pharmaceutique selon la revendication 10, **caractérisée en ce que** ledit cosolvant est présent dans des proportions allant de 7 à 15% en poids.

13. Composition pharmaceutique selon une quelconque des revendications précédentes, **caractérisée en ce qu'**elle comprend en outre au moins une substance de régulation de pH choisie dans le groupe constitué d'acide acétique, trométamol, acide citrique, citrate de sodium, L-arginine, L-lysine, monoéthanolamine, diéthanolamine, triéthanolamine, acide hydrochlrorique, acide tartrique, acide ascorbique et acide phosphorique.

14. Composition pharmaceutique selon la revendication 13, **caractérisée en ce que** ladite substance régulatrice de pH est un acide citrique.

15. Composition pharmaceutique selon une quelconque des revendications précédentes, **caractérisée en ce qu'**elle comprend en outre au moins un agent antioxydant choisi dans le groupe constitué d'EDTA, métabisulfite de sodium et formaldéhyde-sulfoxylate de sodium.

16. Composition pharmaceutique selon une quelconque des revendications précédentes, **caractérisée en ce qu'**elle comprend en outre au moins un agent de conservation choisi dans le groupe constitué d'alcool benzylique, éthanol absolu, propylparaben, phénol, chlorocrésol, chlorure de benzalkonium, butylparaben, méthylparaben, acide benzoïque, alcool phényléthylique et benzoate de sodium.

17. Composition pharmaceutique selon une quelconque des revendications précédentes, **caractérisée en ce qu'**elle comprend en outre au moins un agent modificateur des caractéristiques organoleptiques, choisi dans le groupe constitué d'exhausteurs de goût, colorants et agents aromatisants.

18. Composition pharmaceutique selon une quelconque des revendications précédentes, **caractérisée en ce qu'**elle comprend en outre au moins un agent de complexation choisi dans le groupe constitué d'oxyde de magnésium, chlorure de magnésium, chlorure de calcium et un sel pharmaceutiquement acceptable de cation divalent.

19. Composition pharmaceutique selon une quelconque des revendications précédentes, **caractérisée en ce qu'**elle comprend en outre au moins un agent chélateur choisi dans le groupe constitué d'EDTA, acide citrique et citrate de sodium.

20. Composition pharmaceutique selon une quelconque des revendications précédentes, **caractérisée en ce qu'**elle comprend en outre au moins un adjuvant technologique choisi dans le groupe constitué de polyvinylpyrrolidone, carboxyméthylcellulose et hydroxypropylcellulose.

21. Composition pharmaceutique selon une quelconque des revendications précédentes, **caractérisée en ce qu'**elle comprend en outre au moins un autre ingrédient actif choisi dans le groupe constitué d'un mucolytique, un expectorant, un antitussif, un anti-inflammatoire, un antipyrétique, un bronchodilatateur, un antihistaminique, un bêtabloquant et un antibiotique.

22. Composition pharmaceutique selon la revendication 21, **caractérisée en ce que** ledit autre ingrédient actif est choisi dans le groupe constitué de bromhexine, acétylcystéine, bronvanexine, ambroxol, carbocystéine, guaïfénésine, extraits de plantes ou dérivés, tels que l'hydrate de pinol, *hedera helix, eucaliptus globulus,* Tolù balsam, aspirine, ibuprofène, paracétamol, métamizol, flunixine méglumine, kétoprofène, naproxène, diclofénac, codéine, dextrométorphane, noscapine, dihydrocodéine, dimémorphane, clopérastine, lévodropropizine, fominoben, extrait de Drosera Sp, teinture de Drosera rotundifolia, éphédrine, chlorphénamine, ébastine, loratadine, cétirizine, téofiline, salbutamol, salmétérol, terbutaline, clenbutérol, bambutérol, étamiphylline, carazolol, propanolol, aténolol, gentamicine, amoxicilline, tiamuline, colistine, néomicine, lincomycine, enrofloxacine, ciprofloxacine, norfloxacine, érythromycine, streptomycine, florfénicol, ceftiofur, ivermectine, closantel, lévamisole et chlorsulon.

23. Composition pharmaceutique selon la revendication 22, **caractérisée en ce que** ledit autre ingrédient actif est de la bromhexine.

24. Composition pharmaceutique selon la revendication 22, **caractérisée en ce que** ledit autre ingrédient actif est du paracétamol.

25. Composition pharmaceutique selon une quelconque des revendications précédentes, **caractérisée en ce qu'**elle est administrée par voies orale, parentérale, intranasale, rectale, inhalatoire, transcutanée, topique, intramusculaire, sous-cutanée, intraveineuse, intradermique, intrathécale et intramammaire.

26. Procédé d'obtention d'une composition pharmaceutique, selon une quelconque des revendications précédentes, **caractérisé en ce qu'**il comprend les étapes suivantes :
a) ajout d'au moins un solvant dans un réacteur obtenu en remuant, puis en remuant à nouveau par la suite.
b) Ajout d'un ingrédient actif et, optionnellement, au moins un cosolvant, en continuant à remuer et en établissant la vitesse adéquate jusqu'à dissolution complète.
c) Ajout d'un déshydratant et, optionnellement, au moins un cosolvant, en maintenant le mécanisme de brassage.
d) Ajout du reste des ingrédients jusqu'à dissolution et homogénéisation totales dans lesquelles ledit solvant, ledit ingrédient actif, ledit agent déshydratant et ledit cosolvant sont tels que définis dans les revendications précédentes.

27. Procédé selon la revendication 26, **caractérisé en ce que** ledit reste des ingrédients est choisi dans le groupe constitué d'une substance régulatrice de pH, d'un agent antioxydant, d'un agent de conservation, d'un agent modificateur des caractéristiques organoleptiques, un agent de complexation, un agent chélateur, un adjuvant technologique et un autre ingrédient actif.

28. Procédé selon la revendication 27, **caractérisé en ce que** ledit autre ingrédient actif est choisi parmi le paracétamol et la bromhexine et est ajouté après dissolution dans le cosolvant ou dans une quantité suffisante du solvant principal.

29. Utilisation d'une composition pharmaceutique, selon une quelconque des revendications 1 à 25 pour la fabrication d'un médicament pour la prévention et/ou le traitement d'une pathologie respiratoire, dermatologique, digestive, circulatoire, articulaire ou tissulaire de toute éthologie, pour laquelle les ingrédients actifs de la formule se montrent efficaces.

30. Utilisation d'une composition pharmaceutique selon la revendication 29, **caractérisée en ce que** ladite administration s'adresse à des individus de l'espèce humaine, porcine, bovine, équine, canine, féline ainsi qu'aux oiseaux, rongeurs, reptiles, volailles, oiseaux de compagnie, animaux marins, animaux d'eau douce et poissons d'ornement.

31. Utilisation d'une composition pharmaceutique selon la revendication 29, **caractérisée en ce que** ladite administration s'adresse au bétail et au traitement de fermes piscicoles d'eau douce ou d'eau de mer.

32. Utilisation d'une composition pharmaceutique selon une quelconque des revendications 29 à 31, **caractérisée en ce que** ladite administration est réalisée au moyen d'eau, de dissolution directe, à l'aide de systèmes de distribution automatiques, système de distribution en ligne, agents mécaniques de brassage ou à l'aide de tout autre procédé ou système de distribution acceptable.
